# EUROPEAN PATENT SPECIFICATION

(11) **EP 4 520 375 B1**
(45) Date of publication and mention of the grant of the patent: **08.07.2026**
(21) Application number: 24194972.6
(22) Date of filing: 16.08.2024
(51) Int. Cl.: A61M 16/04

(54) **SPUTUM SUCTION STRUCTURE OF TRACHEOSTOMY TUBE**
SPUTUMABSAUGUNGSSTRUKTUR EINES TRACHEOSTOMIETUBUS
STRUCTURE D'ASPIRATION D'EXPECTORATIONS DE TUBE DE TRACHÉOTOMIE

(30) Priority: 18.08.2023 TW 112131220
(43) Date of publication of application: 12.03.2025
(73) Proprietor: Vitaltec Corporation, Taichung City 42142 (TW)
(72) Inventor: CHIU, Sheng-Yu, 42142 Taichung City (TW)
(74) Representative: 2K Patent Partnerschaft mbB

(56) References cited:
- EP-A1- 2 708 257
- EP-A1- 3 613 454

## Description

### BACKGROUND OF THE DISCLOSURE

### Technical Field

The technical field relates to a sputum suction structure, and more particularly relates to a sputum suction structure of a tracheostomy tube.

### Description of Related Art

A tracheostomy tube is used to perform tracheostomy surgery. The tracheostomy tube is inserted into the patient's neck stoma, and a breathing tube is connected to the connector at the front end of the tracheostomy tube to ensure the patient breathe smoothly.

EP 2708257 A1 discloses a tracheal tube which includes an airway tubular body, an inflatable cuff disposed on the airway tubular body, a suction conduit extending along the airway tubular body to terminate at a suction opening, and a guiding unit disposed on the airway tubular body and adjacent to the suction opening. The guiding unit includes at least one guiding member having a plurality of ribs. Each two adjacent ones of the ribs define a guiding groove that extends toward the suction opening and that terminates at a confluent region in fluid communication with the suction opening so as to guide secretions in the trachea to the suction opening.

Furthermore, after the installation of a tracheostomy tube, the cuff of the tracheostomy tube may abut against the inner wall of the trachea, which cause the patient's sputum to accumulate above the cuff. Additionally, the accumulated sputum increases the risk of infection and respiratory obstruction, thus sputum suction is required. The current sputum suction process involves attaching a suction tube to the outer side of the cuff to suction the fixed point. Since the end of the suction tube is positioned away from the upper edge of the cuff during installation, the secretions and sputum accumulated on the upper edge of the cuff may not be extracted, and the sputum around the periphery of the suction tube is difficult to be removed. This results in the sputum accumulation and retained above the cuff.

In view of the above drawbacks, the inventor proposes this disclosure based on his expert knowledge and elaborate researches in order to solve the problems of related art

### SUMMARY OF THE INVENTION

This application provides a sputum suction structure of a tracheostomy tube to suction the sputum at the upper edge of the cuff over a wider range, and removing the sputum at the upper edge of the cuff is achieved efficiently.

The sputum suction structure of a tracheostomy tube according to the invention is defined in appended claim 1. Advantageous embodiments are the subject of the dependent claims.

In comparison with the related art, the sputum suction structure of the tracheostomy tube of this disclosure includes a suction pad. Since the suction pad covers the insertion tube and is adjacent to the top of the cuff outside. Additionally, since the suction pad includes the sputum suction port equipping with side suction opening on two sides thereof; thus, the tracheostomy tube does not need to attach the suction tube outside of the cuff. Therefore, the sputum above the cuff is sucked into the suction space from the bottom side of the suction pad and the side suction openings on two sides thereof by the negative pressure suction of the suction tube, then enters the sputum suction port, and finally flows into the suction tube and is pumped out, thus the sputum above the cuff may be suctioned in a wider range. Therefore, the sputum above the cuff may be removed more effectively to increase the practicality of this disclosure.

### BRIEF DESCRIPTION OF THE DRAWINGS

The features of the disclosure believed to be novel are set forth with particularity in the appended claims. The disclosure itself, however, may be best understood by reference to the following detailed description of the disclosure, which describes a number of exemplary embodiments of the disclosure, taken in conjunction with the accompanying drawings, in which:
FIG. 1 depicts a perspective schematic view of the tracheostomy tube according to this disclosure.
FIG. 2 depicts an assembly schematic view of the suction pad according to this disclosure.
FIG. 3 depicts a perspective schematic view of the suction pad according to this disclosure.
FIG. 4 to FIG. 6 depict perspective schematic views of the tracheostomy tube from different sides according to this disclosure.
FIG. 7 depicts the combination schematic view of the suction pad and the suction tube according to this disclosure.
FIG. 8 is a schematic view showing the flow direction of sputum during suction according to this disclosure.
FIG. 9 is a schematic view depicting the tracheostomy tube suctioning secretions while the patient is sitting or standing.
FIG. 10 is a schematic view depicting the tracheostomy tube suctioning secretions while the patient is lying down.
FIG. 11 depicts a perspective schematic view of another embodiment of the suction pad according to this disclosure.
FIG. 12 depicts a perspective schematic view of still another embodiment of the suction pad according to this disclosure.

### DETAILED DESCRIPTION

The technical contents of this disclosure will become apparent with the detailed description of embodiments accompanied with the illustration of related drawings as follows. It is intended that the embodiments and drawings disclosed herein are to be considered illustrative rather than restrictive.

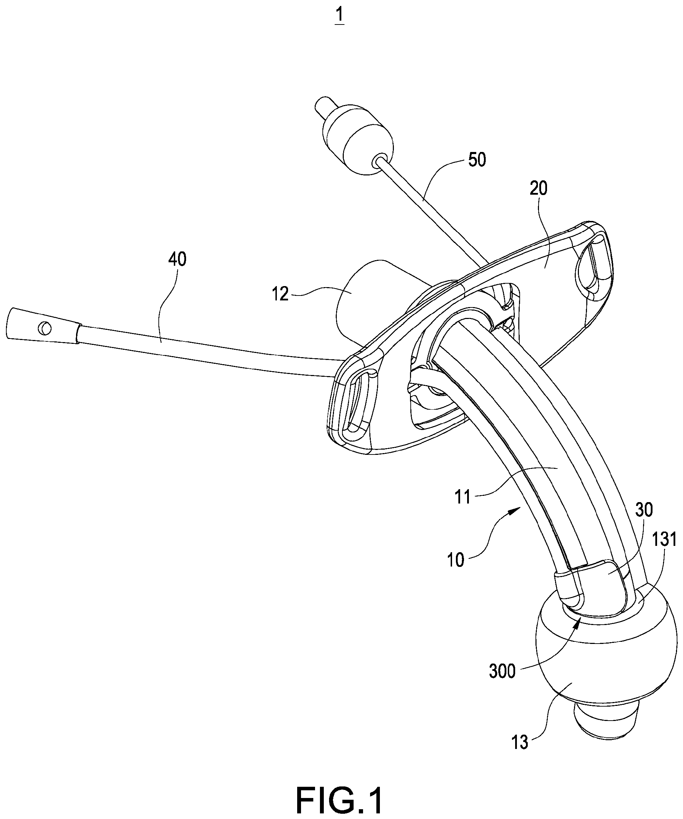

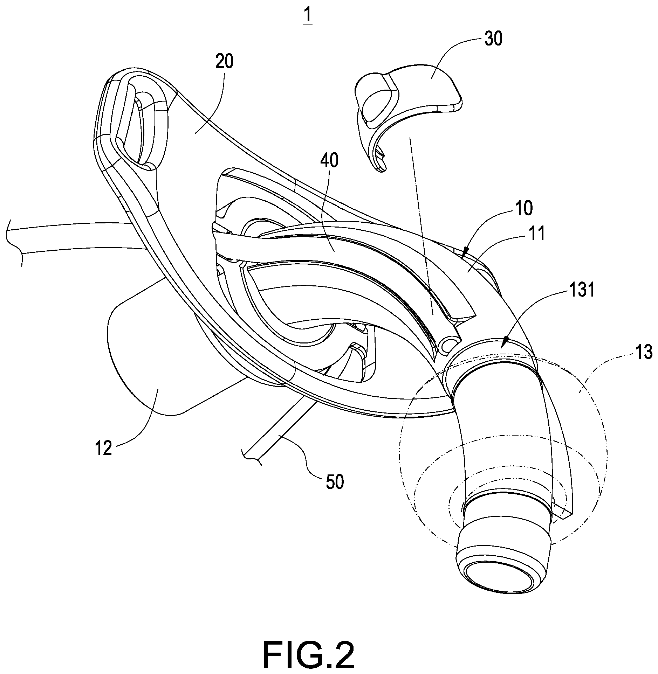

Please refer to FIG.1 and FIG. 2, which depict a perspective schematic view of the tracheostomy tube according to this disclosure and an assembly schematic view of the suction pad according to this disclosure. The sputum suction structure of a tracheostomy tube 1 includes an insertion tube 10, a movable baffle 20, a suction pad 30, and a suction tube 40. The movable baffle 20 is placed outside the insertion tube 10. The suction pad 30 is combined on the insertion tube 10. The suction tube 40 is inserted in the suction pas 30 and protrudes from the movable baffle 20 to connect a suction device (not shown). Accordingly, the suction tube 40 may extract sputum accumulated at the bottom of the suction pad 30.

Specifically, the insertion tube 10 includes an insertion section 11, a connection section 12 connected to the insertion section 11, and a cuff 13 disposed on the insertion section 11. In this embodiment, the tracheostomy tube 1 further includes an inflation tube 50. The inflation tube 50 is inserted in the insertion section 11 and communicates with the cuff 13 to connect an air supply device (not shown). Thus, external air may flow into the cuff 13 from the inflation tube 50 to make the cuff 13 to inflate to expand the patient's trachea.

The movable baffle 20 is positioned between the insertion section 11 and the connection section 12. In some embodiments, the movable baffle 20 is placed outside the stoma of the patient's throat to position the insertion tube 10.

Furthermore, the suction pad 30 is located on the upper edge 131 of the cuff 13 facing the insertion section 11 and encloses at least a part of the outer periphery of the insertion section 11. The suction tube 40 is inserted in one side of the suction pad 30. Additionally, the suction tube 40 extends and protrudes from the movable baffle 20. A more detailed description of the combination structure of the suction pad 30 and the suction tube 40 is as follows.

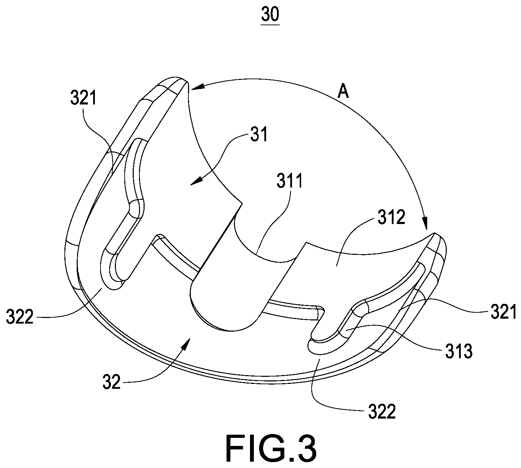

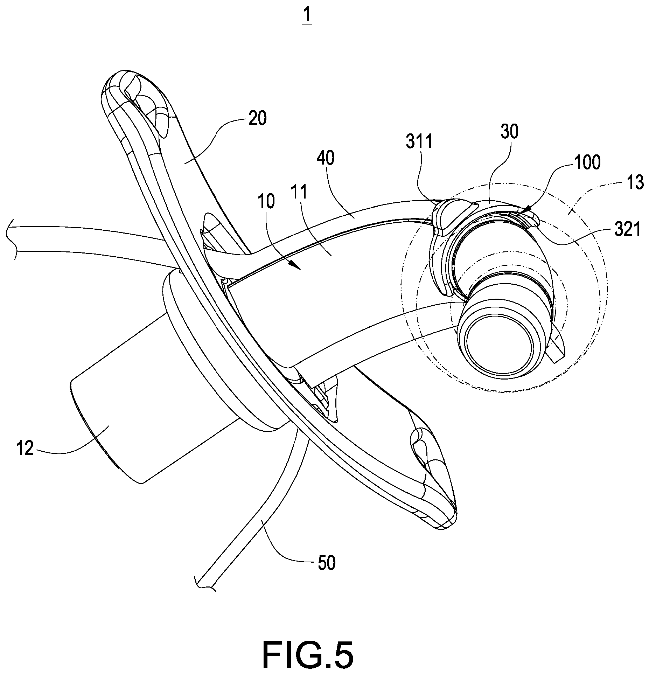

Please further refer to FIG. 3 to FIG. 6, which depict a perspective schematic view of the suction pad according to this disclosure and two perspective schematic views of the tracheostomy tube from different sides according to this disclosure. In this embodiment, the suction pad 30 includes a semicircular ring plate. The suction pad 30 includes a coupling part 31 disposed on one side thereof and a suction part 32 disposed on another side thereof. The coupling part 31 is tightly attached to the outer periphery of the insertion section 11 and includes a sputum suction port 311 communicating with the suction part 32.

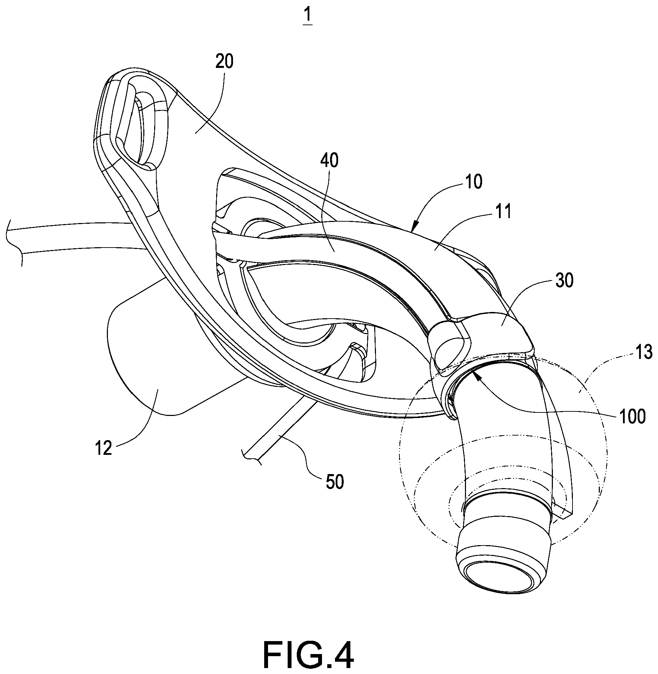

It is worth of noticing that an extension angle A is defined on the suction pad 30, and the extension angle A is less than or equal to 180 degrees. Additionally, a gap 300 (see FIG. 1) is defined between the side edge and the upper edge 131 of the suction pad 30. Therefore, a suction space 100 (see FIG. 4) is defined between the suction part 32 and the upper edge 131 of the cuff 13, and the suction part 32 includes a side suction opening 321 communicating with the suction space 100 on both sides thereof.

In more detail, a step portion is configured between the coupling part 31 and the suction part 32. Moreover, the coupling part 31 includes a convex binding surface 312 and a plurality of blocking ribs 313 connected to the convex binding surface 312. The blocking ribs 313 are located on two sides of the suction pad 30 and the side suction opening 321 is defined on the outer sides of the blocking ribs 313.

Furthermore, each blocking rib 313 extends from the convex binding surface 312 and a side flow channel 322 is defined between each blocking rib 313 and the side edge of the suction pad 30. It should be noted that the side flow channel 322 is configured to restrict the path for sputum to enter the sputum suction port 311. Thus, the sputum may be concentrated from the side flow channel 322 into the sputum suction port 311 under a certain suction force.

Further, since a gap exists between the side suction opening 321 of the suction part 32 and the sputum suction port 311, negative pressure generated during suction may cause the suction part 32 to deform. Additionally, the arrangement of the blocking ribs 313 may help prevent such deformation of the suction part 32 due to suction. Therefore, the problem of ineffective suction may be avoided.

In one embodiment of this application, the sputum suction port 311 extends from the coupling part 31 to the suction part 32. The sputum suction port 311 is structured as a concave arc surface located on the inner wall of the suction pad 30 facing the insertion section 11. The suction pad 30 includes a convex arc surface located on the outer surface thereof away from the insertion section 11.

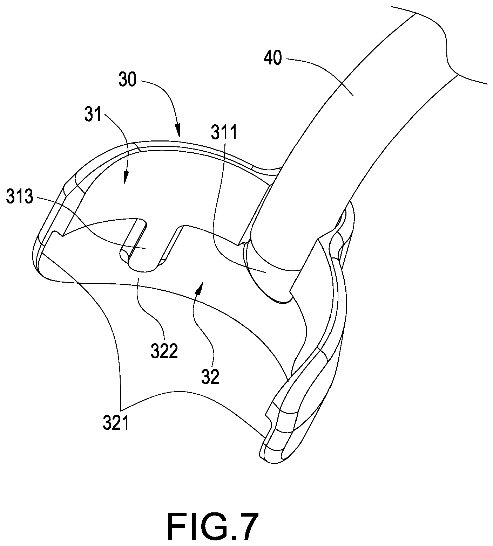

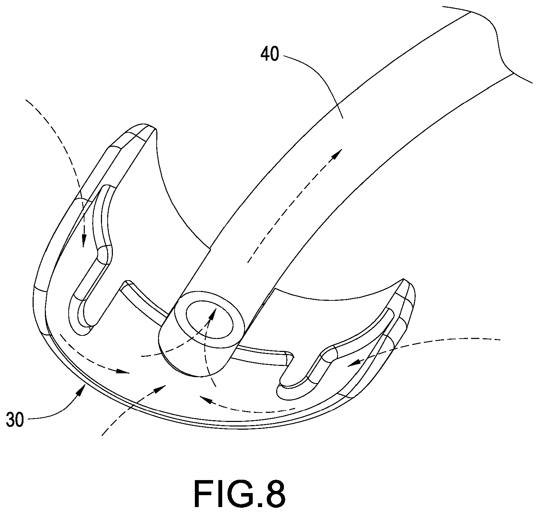

Please refer to FIG. 7 and FIG. 8, which depicts the combination schematic view of the suction pad and the sputum suction tube according to this disclosure and a schematic view showing the flow direction of sputum during suction according to this disclosure. The suction pad 30 includes a coupling part 31 and a suction part 32. The suction pad 30 is fixed on the insertion tube 10 through the coupling part 31 being tightly attached to the outer periphery of the insertion section 11, and the coupling part 31 includes a sputum suction port 311 communicating with the suction part 32. Furthermore, one end of the suction tube 40 is inserted in the sputum suction port 311, and the other end of the suction tube 40 is extended and protrudes from the outer side of the movable baffle 20 ( see FIG.1 ). Additionally, the coupling part 31 includes a plurality of blocking ribs 313. The two outer sides of the blocking ribs 313 are side suction openings 321. A side flow channel 322 is defined between each blocking rib 313 and the side edge of the suction pad 30.

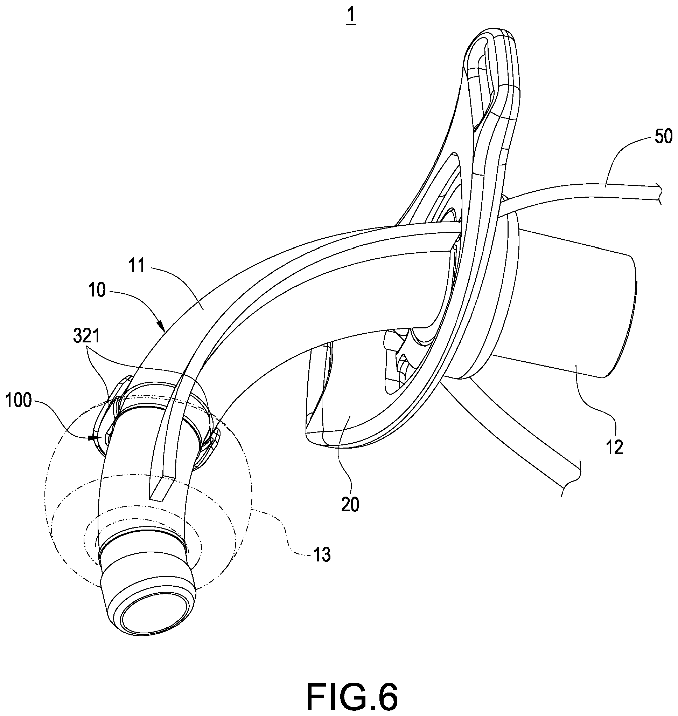

During the sputum suction operation, the suction device is activated to generate negative pressure and suck the accumulated sputum above the cuff 13 through the suction tube 40 (see FIG. 1). Furthermore, the sputum retained above the cuff 13 is subjected to the negative pressure suction of the suction tube 40 and enters the suction part 32 from the bottom side of the suction pad 30 and the two side suction openings 321 of the suction pad 30 (see FIG.6), and then flows through the side flow channel 322 and enters the sputum suction port 311, and finally flows into the suction tube 40 and is pumped out.

It is worth noticing that since the sputum above the cuff 13 may enter the suction part 32 from the side suction openings 321 on two sides of the suction pad 30, the sputum above the cuff 13 may be suctioned in a wider range. Therefore, it may more effectively and reliably to remove spectrum above the cuff.

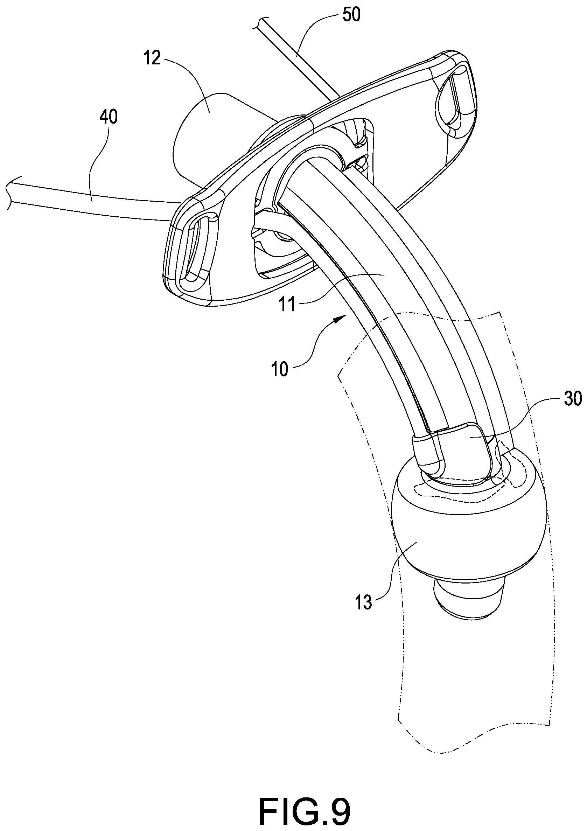

Please refer to FIG. 9, which is a schematic view depicting the tracheostomy tube suctioning secretions while the patient is sitting or standing. In the tracheostomy tube of this disclosure, secretions are accumulate above the cuff 13 and located at the bottom side of the suction pad 30 while the patient is in a sitting position. During the sputum suction operation, secretions enters the suction part 32 from the bottom side of the suction pad 30 (see FIG. 6), and then flows through the side flow channel 322 into the suction port 311, and finally flows into the suction tube 40 and is pumped out (see FIGs. 7 and 8).

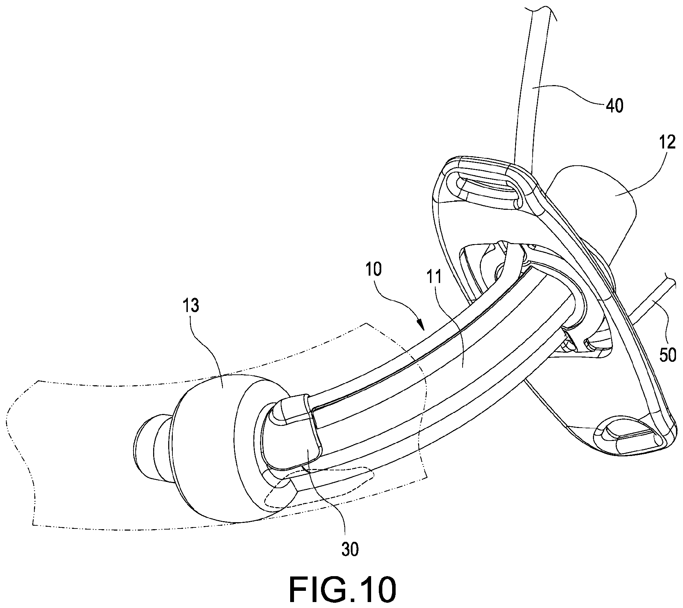

Please further refer to FIG. 10, which is a schematic view depicting the tracheostomy tube suctioning secretions while the patient is lying down. In the tracheostomy tube of this disclosure, secretions is accumulated above the cuff 13 and located on one side of the suction pad 30. During the sputum suction operation, under the action of the negative pressure of the sputum suction tube 40, secretions enters the suction part 32 from the side suction opening 321 of the suction pad 30 (see FIG. 7), and then flows into the suction tube 40 and is pumped out (see also FIG. 8).

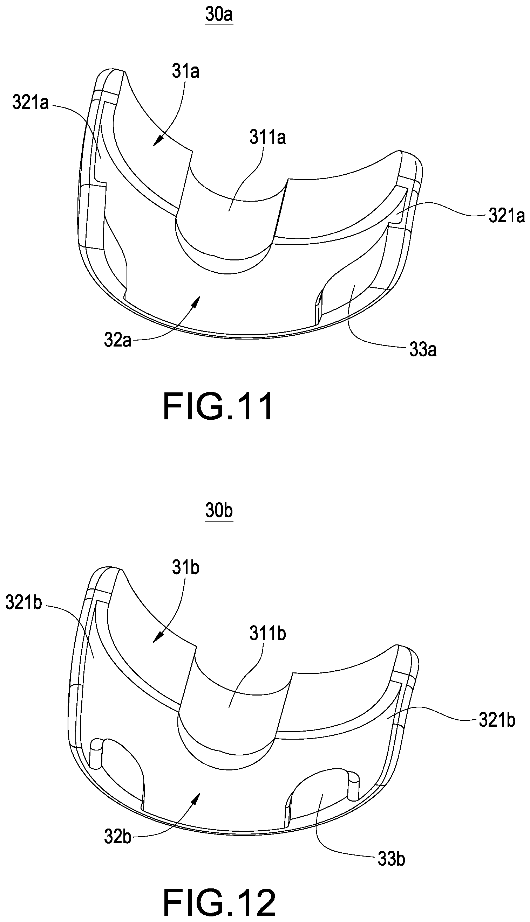

Please refer to FIG. 11, which depicts a perspective schematic view of another embodiment of the suction pad according to this disclosure. In this embodiment, the suction pad 30a includes a coupling part 31a located on one side and a suction part 32a located on another side thereof oppositely. The coupling part 31a includes a sputum suction port 311a communicating with the suction part 32a in the middle position. Moreover, a step portion is defined between the coupling part 31a and the suction part 32a.

The difference between this embodiment and the previous embodiment is that the suction pad 30a is formed with a bump 33a on two sides of the bottom edge of the suction part 32a. In addition, a suction port 321a communicating with the suction part 32a is defined between the coupling portion 31a and the bump 33a.

Please refer to FIG. 12, which depicts a perspective schematic view of still another embodiment of the suction pad according to this disclosure. In this embodiment, the suction pad 30b includes a coupling part 31b disposed on one side and a suction part 32b disposed on another thereof side thereof. The coupling part 31b includes a sputum suction port 311b communicating with the suction part 32b in the middle position. Moreover, a step portion is defined between the coupling part 31b and the suction part 32b.

The difference between this embodiment and the previous embodiment is that the suction pad 30b includes bumps 33b located on the bottom edge of the suction part 32b and distanced from two sides of the suction part 32b. Additionally, a side suction opening 321b is defined between the coupling part 31b and the bump 33b.

## Claims

1. A sputum suction structure of a tracheostomy tube, the sputum suction structure comprising:
an insertion tube (10), comprising an insertion section (11), a connection section (12) connected to the insertion section (11), and a cuff (13) disposed on the insertion section (11);
a movable baffle (20), positioned between the insertion section (11) and the connection section (12);
a suction pad (30), located on an upper edge (131) of the cuff (13) facing the insertion section (11) and enclosing at least a part of an outer periphery of the insertion section (11), comprising a coupling part (31) disposed on one side thereof and a suction part (32) disposed on another side thereof, wherein the coupling part (31) is tightly attached to the outer periphery of the insertion section (11) and comprises a sputum suction port (311) communicating with the suction part (32);
**characterized in that** a suction space (100) is defined between the suction part (32) and the upper edge (131) of the cuff (13), and the suction part (32) comprises a side suction opening (321) communicating with the suction space (100) and defined on two sides thereof respectively; and
a suction tube (40), one end thereof being inserted in the sputum suction port (311) and another end thereof extending and protruding from an outer side of the movable baffle (20);
wherein a gap (300) is reserved between a side edge of the suction pad (30) and the upper edge (131) of the cuff (13).

2. The sputum suction structure according to claim 1, further comprising an inflation tube (50), wherein the inflation tube (50) is inserted in the insertion section (11) and communicates with the cuff (13).

3. The sputum suction structure according to claim 1, wherein the sputum suction port (311) is structured as a concave arc surface located on an inner wall of the suction pad (30) facing the insertion section (11), and the suction pad (30) comprises a convex arc surface located on an outer surface thereof away from the insertion section (11).

4. The sputum suction structure according to claim 1, wherein the sputum suction port (311) extends from the coupling part (31) to the suction part (32).

5. The sputum suction structure according to claim 1, wherein the suction pad (30) comprises a semicircular ring plate.

6. The sputum suction structure according to claim 5, wherein an extension angle (A) is defined on the suction pad (30), and the extension angle (A) is less than or equal to 180 degrees.

7. The sputum suction structure according to claim 1, wherein a step portion is disposed between the coupling part (31) and the suction part (32).

8. The sputum suction structure according to claim 1, wherein the coupling part (31) comprises a convex binding surface (312) and a plurality of blocking ribs (313) connected to the convex binding surface (312), and the blocking ribs (313) are located on two sides of the suction pad (30), and the side suction opening (321) is defined on outer sides of the blocking ribs (313).

9. The sputum suction structure according to claim 8, wherein each blocking rib (313) extends from the convex binding surface (312), and a side flow channel (322) is defined between each blocking rib (313) and a side edge of the suction pad (30).

10. The sputum suction structure according to claim 1, wherein the suction pad (30a) comprises a bump (33a) located on two sides of a bottom edge of the suction part (32a), and the side suction opening (321a) is defined between the coupling part (31a) and the bump (33a).

11. The sputum suction structure according to claim 1, wherein the suction pad (30b) comprises bumps (33b) located on a bottom edge of the suction part (32b) and distanced from two sides of the suction part (32b), and the side suction opening (321b) is defined between the coupling part (31b) and the bump (33b).

## Patentansprüche

1. Sputumabsaugungsstruktur für einen Tracheostomiietubus, wobei die Sputumabsaugungsstruktur umfasst:
ein Einführungsrohr (10), das einen Einführabschnitt (11), einen mit dem Einführabschnitt (11) verbundenen Anschlussabschnitt (12) und eine am Einführabschnitt (11) angeordnete Manschette (13) umfasst;
ein beweglicher Deflektor (20), der zwischen dem Einführabschnitt (11) und dem Anschlussabschnitt (12) positioniert ist;
einem Saugpolster (30), das sich an einer der dem Einführabschnitt (11) zugewandten oberen Kante (131) der Manschette (13) befindet und mindestens einen Teil des Außenumfangs des Einführabschnitts (11) umschließt, das einen an einer Seite davon angeordeten Kopplungsteil (31) und ein an einer anderen Seite davon angeordnetes Saugteil (32) umfasst, wobei der Kopplungsteil (31) fest an dem Außenumfang des Einführabschnitts (11) befestigt ist und eine mit dem Saugteil (32) in Verbindung stehende Sputumabsaugöffnung (311) umfasst;
**dadurch gekennzeichnet, dass** zwischen dem Saugteil (32) und der oberen Kante (131) der Manschette (13) ein Saugraum (100) definiert ist und das Saugteil (32) eine seitliche Saugöffnung (321) aufweist, die mit dem Saugraum (100) in Verbindung steht und an zwei Seiten desselben jeweils definiert ist; und
ein Saugrohr (40), dessen eines Ende in die Sputum-Sputumabsaugöffnung (311) eingeführt ist und dessen anderes Ende sich von einer Außenseite des beweglichen Deflektors (20) erstreckt und aus diesem herausragt;
wobei zwischen einer Seitenkante des Saugpolsters (30) und der oberen Kante (131) der Manschette (13) ein Spalt (300) vorgesehen ist.

2. Sputumumabsaugungsstruktur nach Anspruch 1, ferner umfassend ein Aufblasrohr (50), wobei das Aufblasrohr (50) in den Einführabschnitt (11) eingeführt ist und mit der Manschette (13) in Verbindung steht.

3. Sputumabsaugungsstruktur nach Anspruch 1, wobei die Sputumabsaugöffnung (311) als konkave Krumme ausgebildet ist, die sich an einer dem Einführabschnitt (11) zugewandten Innenwand des Saugpolsterns (30) befindet, und das Saugpolster (30) eine konvexe Krumme aufweist, die sich an einer vom Einführabschnitt (11) abgewandten Außenfläche desselben befindet.

4. Sputumabsaugungsstruktur nach Anspruch 1, wobei sich die Sputumabsaugöffnung (311) vom Kopplungsteil (31) zum Saugteil (32) erstreckt.

5. Sputumumabsaugungsstruktur nach Anspruch 1, wobei das Saugpolster (30) eine halbkreisförmige Ringplatte umfasst.

6. Sputumumabsaugungsstruktur nach Anspruch 5, wobei an dem Saugpolster (30) ein Ausdehnungswinkel (A) definiert ist und der Ausdehnungswinkel (A) kleiner oder gleich 180 Grad ist.

7. Sputumumabsaugungsstruktur nach Anspruch 1, wobei zwischen dem Kopplungsteil (31) und dem Saugteil (32) ein Stufenabschnitt angeordnet ist.

8. Sputumabsaugungsstruktur nach Anspruch 1, wobei das Kopplungsteil (31) eine konvexe Bindfläche (312) und eine Vielzahl von mit der konvexen Bindfläche (312) verbundenen Blockierrippen (313) umfasst, und die Blockierrippen (313) an zwei Seiten des Saugpolsters (30) angeordnet sind und die seitliche Saugöffnung (321) an den Außenseiten der Blockierrippen (313) definiert ist.

9. Sputumumabsaugungsstruktur nach Anspruch 8, wobei sich jede Blockierrippe (313) von der konvexen Bindfläche (312) erstreckt und ein seitlicher Fließkanal (322) zwischen jeder Blockierrippe (313) und einer Seitenkante des Saugpolsers (30) definiert ist.

10. Sputumumabsaugungsstruktur nach Anspruch 1, wobei das Saugpolster (30a) einen Vorsprung (33a) aufweist, der an zwei Seiten einer Unterkante des Saugteils (32a) angeordnet ist, und die seitliche Saugöffnung (321a) zwischen dem Kopplungsteil (31a) und dem Vorsprung (33a) definiert ist.

11. Sputumumabsaugungsstruktur nach Anspruch 1, wobei das Saugpolster (30b) Vorsprünge (33b) aufweist, die an einer Unterkante des Saugteils (32b) angeordnet und von beiden Seiten des Saugteils (32b) beabstandet sind, und die seitliche Saugöffnung (321b) zwischen dem Kopplungsteil (31b) und dem Vorsprung (33b) definiert ist.

## Revendications

1. Une structure d'aspiration des expectorations pour un tube de trachéotomie, ladite structure d'aspiration des expectorations comprenant :
un tube d'insertion (10) comprenant une partie d'insertion (11), une partie de raccordement (12) reliée à la partie d'insertion (11) et un manchon (13) disposé sur la partie d'insertion (11) ;
un déflecteur mobile (20) positionné entre la partie d'insertion (11) et la partie de raccordement (12) ;
un coussin d'aspiration (30) situé sur l'un des bords supérieurs de la manchette (13) tourné vers la partie d'introduction (11) (131) du manchon (13) et qui entoure au moins une partie de la périphérie extérieure de la partie d'introduction (11), qui comprend une partie d'accouplement (31) disposée sur un côté de celle-ci et une partie d'aspiration (32) disposée sur un autre côté de celle-ci, la partie d'accouplement (31) étant solidement fixée à la périphérie extérieure de la partie d'insertion (11) et comprenant une ouverture d'aspiration des expectorations (311) communiquant avec la partie d'aspiration (32) ;
**caractérisé en ce qu'**un espace d'aspiration (100) est défini entre la partie d'aspiration (32) et le bord supérieur (131) du manchon (13) et **en ce que** la partie d'aspiration (32) comporte une ouverture d'aspiration latérale (321) qui communique avec l'espace d'aspiration (100) et qui est définie sur deux côtés de celui-ci ; et
un tube d'aspiration (40) dont une extrémité est introduite dans l'ouverture d'aspiration des expectorations (311) et dont l'autre extrémité s'étend depuis une face extérieure du déflecteur mobile (20) et dépasse de celui-ci ;
un espace (300) étant prévu entre un bord latéral du coussin d'aspiration (30) et le bord supérieur (131) de la manchette (13).

2. La structure d'aspiration des expectorations selon la revendication 1, comprenant en outre un tube de gonflage (50), le tube de gonflage (50) étant inséré dans la partie d'insertion (11) et communiquant avec le manchon (13).

3. La structure d'aspiration des expectorations selon la revendication 1, dans laquelle l'ouverture d'aspiration des expectorations (311) est formée comme une courbe concave située sur une paroi intérieure du coussin d'aspiration (30) tournée vers la partie d'insertion (11), et le coussin d'aspiration (30) présente une courbe convexe située sur une surface extérieure de celui-ci opposée à la partie d'insertion (11).

4. La structure d'aspiration des expectorations selon la revendication 1, dans laquelle l'ouverture d'aspiration des expectorations (311) s'étend de la partie d'accouplement (31) à la partie d'aspiration (32).

5. La structure d'aspiration de crachats selon la revendication 1, dans laquelle le coussin d'aspiration (30) comprend une plaque annulaire semi-circulaire.

6. La structure d'aspiration de crachats selon la revendication 5, dans laquelle un angle d'extension (A) est défini sur le coussin d'aspiration (30) et l'angle d'extension (A) est inférieur ou égal à 180 degrés.

7. La structure d'aspiration de mucosités selon la revendication 1, dans laquelle une partie en gradins est disposée entre la partie d'accouplement (31) et la partie d'aspiration (32).

8. La structure d'aspiration de mucosités selon la revendication 1, dans laquelle la partie d'accouplement (31) comprend une surface de liaison convexe (312) et une pluralité de nervures de blocage (313) reliées à la surface de liaison convexe (312), et les nervures de blocage (313) sont disposées sur deux côtés du coussin d'aspiration (30) et l'ouverture d'aspiration latérale (321) est définie sur les côtés extérieurs des nervures de blocage (313).

9. La structure d'aspiration d'expectorations selon la revendication 8, dans laquelle chaque nervure de blocage (313) s'étend à partir de la surface de liaison convexe (312) et un canal d'écoulement latéral (322) est défini entre chaque nervure de blocage (313) et un bord latéral du coussin d'aspiration (30).

10. La structure d'aspiration d'expectorations selon la revendication 1, dans laquelle le coussin d'aspiration (30a) comporte une saillie (33a) disposée sur deux côtés d'un bord inférieur de la partie d'aspiration (32a), et l'ouverture d'aspiration latérale (321a) est définie entre la partie d'accouplement (31a) et la saillie (33a).

11. La structure d'aspiration des expectorations selon la revendication 1, dans laquelle le coussin d'aspiration (30b) comporte des saillies (33b) qui sont disposées sur un bord inférieur de la partie d'aspiration (32b) et espacées des deux côtés de la partie d'aspiration (32b), et l'ouverture d'aspiration latérale (321b) est définie entre la partie d'accouplement (31b) et la saillie (33b).
